# EUROPEAN PATENT SPECIFICATION

(11) **EP 1 442 765 B1**
(45) Date of publication and mention of the grant of the patent: **29.08.2012**
(21) Application number: 02765597.6
(22) Date of filing: 19.09.2002
(51) Int. Cl.: A61M 16/04, A61M 25/00

(54) **IMPLEMENT FOR ASSISTING INFLATION OF MEDICAL IMPLEMENT WITH CUFF; AND BRONCHUS CLOSING IMPLEMENT WITH THE IMPLEMENT**
MITTEL ZUR UNTERSTÜTZUNG DER INFLATION EINER MEDIZINISCHEN VORRICHTUNG MIT MANSCHETTE; SOWIE BROCHUS-VERSCHLUSS-VORRICHTUNG MIT DEM MITTEL
DISPOSITIF D'AIDE AU GONFLAGE D'UN DISPOSITIF MEDICAL A MANCHON, ET DISPOSITIF DE FERMETURE BRONCHIQUE POURVU DE CE DISPOSITIF

(30) Priority: 16.10.2001 JP 2001318513
(43) Date of publication of application: 04.08.2004
(73) Proprietor: Daiken Iki Kabushiki Kaisha, Osaka-shi, Osaka 540-0036 (JP)
(72) Inventor: YAMADA, Keiichi, Sakai-shi, Osaka 590-0101 (JP); TAKASHINA, Masaki, Minoo-shi, Osaka 562-0001 (JP)
(74) Representative: Jenkins, Peter David
(86) International application number: PCT/JP2002/009596
(87) International publication number: WO 2003/033063

(56) References cited:
- DE-A1- 2 538 268
- FR-A1- 2 713 938
- JP-B2- 6 098 191
- JP-Y1- 45 001 676
- US-A- 4 159 722
- US-A- 4 248 222
- US-A- 4 501 273
- US-A- 4 501 273
- US-A- 4 552 558
- US-A- 4 649 914
- US-A- 4 649 914
- US-A- 4 856 510
- US-A- 4 856 510

## Description

### TECHNICAL FIELD

The present invention relates to a medical implement with cuff, and more specifically to an inflation assisting implement for inflating the cuff, and a bronchus closing implement equipped with the inflation assisting implement.

### BACKGROUND ART

Heretofore, there has been well known in the art a technique of closing an air or fluid passage of a living body with use of a medical implement with cuff (balloon). For instance, an endotracheal tube employed in artificial respiration is used in a state that an end of the tube is inserted into the mouth of a patient (via oral) or into the nostril of the patient (via nasal), and the other end thereof is connected with a respirator. When the cuff at the endotracheal tube is inflated inside the trachea, the air passages below the trachea (namely, the lungs) are closed, whereby forcible respiration is initiated by the respirator communicated with the endotracheal tube.

As another example of the medical implement with cuff (balloon), there is known a balloon catheter used for pulmonary artery. This catheter is inserted into a blood vessel of a patient by way of an appropriate blood vessel such as a carotid artery, and is conveyed to the pulmonary artery of the patient by the blood flow. When the catheter reaches a target site of the patient's blood vessel, the balloon is inflated, so that an operator can measure a so-called "capillary wedge pressure" representing the blood flow into the lung, by the sensor equipped at the tip of the catheter.

The above medical implement with cuff (balloon) blocks an air or fluid passage of the patient by inflation of the cuff (balloon). The excessive inflation of the cuff (balloon) may likely to damage the tracheal mucosa of the patient due to a stress exerted to the mucosa in the case where the endotracheal tube is used, or damage the blood vessel in the case where the balloon catheter for pulmonary artery is used.

Japanese Unexamined Patent Publication No. HEI 10-272184 proposes an implement for stabilizing the cuff pressure in securing an air passage. In this publication, a rubber balloon is provided in an intake port of the cuff for inflating the cuff in an attempt to solve the above problem residing in the conventional endotracheal tube. The rubber balloon has such a configuration that a desired pressure is generated by supplying the air of a predetermined volume into the rubber balloon. As the balloon is inflated by supply of the air, the cuff is also inflated, with the result that both the cuff and the rubber balloon are inflated, and brought to a balanced state. In this arrangement, there is no likelihood that the cuff is inflated beyond the pressure of the rubber balloon because the inner pressure of the cuff is controlled to such a level as not to damage the tracheal mucosa, thereby preventing damage of the tracheal mucosa.

Further, Japanese Examined Patent Publication No. HEI 06-098191 discloses an implement for keeping patients from rupture of blood vessels under use of a catheter balloon in an attempt to solve the problem residing in the conventional balloon catheter for pulmonary artery. In the publication, the balloon catheter for pulmonary artery is equipped with a gas release/safety member in an inlet (air supply port) thereof for inflating the balloon, and an inlet socket. When the air is supplied into the balloon through the inlet socket by an injection tube, the balloon is inflated as far as the balloon is in a normal inflatable condition. However, if the balloon is located in a relatively narrow blood vessel where its inflation is obstructed, the balloon cannot inflate, with the result that the inner pressure of the implement may be undesirably raised. In the arrangement of the publication, the gas release/safety member is inflated while keeping the pressure of the balloon unchanged, if the inner pressure of the implement exceeds a predetermined pressure. In this way, the implement makes it possible to prevent rupture of the blood vessel by releasing the excessive pressure into the gas release/safety member while keeping the pressure of the balloon unchanged, if the inner pressure of the implement exceeds the predetermined pressure.

As mentioned above, the conventional medical implements with cuff provide various measures, but yet the following drawbacks are unavoidable.

Since the implement disclosed in Japanese Unexamined Patent Publication No. HEI 10-272184 employs a technique of keeping pressure balance between the cuff and the rubber balloon, it is required to use the rubber balloon in such a manner that the pressure of the rubber balloon is controlled so as not to cause rupture of the blood vessel, if the implement is used as a medical implement with cuff (balloon) for blocking the blood vessel. Since the types and sizes of cuffs (balloons) used in the medical implements are numerous, it is difficult for operators to manipulate these variety of medical implements with cuff (balloon), thereby failing to provide versatile use of the implement.

Further, the medical implement with cuff (balloon) disclosed in Japanese Examined Patent Publication No. HEI 06-098191 has poor operability because an operator has to manipulate the injection tube with both of his or her hands in supplying the air through the injection tube. Also, it is difficult to finely adjust the air supply amount into the balloon. Further, the implement is so designed that the gas release/safety member is actuated under the condition that the inner pressure of the implement exceeds a predetermined pressure. In this arrangement, if, for example, the implement is used as a medical implement with cuff (balloon) for blocking an air passage of a patient, it is required to change the construction of the gas release/safety member in such a manner that an excessive pressure which may damage the tracheal mucosa of the patient due to a stress exerted thereto may not be generated. Thus, the implement disclosed in this publication also fails to provide versatile use.

US4649914 discloses a tracheal tube assembly including a dual balloon system upstream from a tracheal cuff and located externally of a patient. Air may be injected through a one-way valve into an innermost balloon which is of relatively low compliance and which passively expands a higher compliance outer balloon. A control valve is openable to selectively interconnect the inner balloon with the outer balloon via a high resistance inlet and with the tracheal cuff via a low resistance inlet so that the tracheal cuff is preferentially inflated.

US4856510 discloses a tracheal tube assembly including an inflator for a cuff that has serially arranged pilot and control balloons, with the control balloon having a higher compliance than the pilot balloon and being connected directly to an inflation tube for a cuff. Check valves are mounted in a support tube extending through the balloons.

US4501273 discloses an endotracheal tube that is encircled by an inflatable cuff, to which cuff is connected a tubule that extends towards an opposite end of the endotracheal tube and into a valve housing having an air inlet bore. The bore includes a valve that normally closes off the inlet, and means for opening the valve to admit air under pressure or to release it therefrom to inflate and deflate the cuff.

In view of the above problems residing in the conventional medical implements with cuff (balloon), an object of the present invention is to provide a cuff inflation assisting implement having simplified operability that enables to prevent an excessive inflation of a cuff (balloon) in blocking the air or fluid passage of a living body by the cuff (balloon), and is usable in various medical implements with cuff (balloon), as well as a bronchus closing implement with the inflation assisting implement.

### DISCLOSURE OF THE INVENTION

One aspect of the present invention provides an inflation assisting implement as defined in claim 1.

In that arrangement, when a healthcare worker injects a fluid with an injection tube into the inflation assisting implement through the check valve, the fluid is temporarily stored in the cuff inflating means. In this state, the connecting part of the implement is connected with an injection port of the medical implement with cuff (balloon). When the stop valve is opened, the fluid in the cuff inflating means is fed to the cuff (balloon) with its flow rate being regulated by the regulating means. This arrangement enables the healthcare worker to inject the fluid into the cuff with a simplified operation. Further, since the flow rate is adjusted by the regulating means, excessive inflation of the cuff (balloon) is prevented.

Preferably, the cuff inflating means includes a retractable container, and a cover member which protects the retractable container and regulates a chargeable amount of the liquid in the retractable container.

In the above arrangement, since the cuff inflating means is comprised of the retractable container, the fluid injected through the check valve is temporarily stored in the retractable container. When the stop valve is opened, the fluid flows out the retractable container due to a contraction force of the retractable container via the regulating means. Furthermore, since the cuff inflating means has the cover member, the retractable container is protected from external influence. Also, since the flow rate of the fluid to be charged into the retractable container is controlled, excessive injection of the fluid into the cuff (balloon) is prevented.

The cuff inflating means may include a cylindrical container, a piston which is slidably movable in the cylindrical container in such a manner that an expandable and contractible fluid storing chamber is defined in the cylindrical container, and urging means which urges the piston in such a direction as to reduce the volume of the fluid storing chamber.

In the above arrangement, the cuff inflating means is comprised of the cylindrical container, the piston which is slidably movable in the cylindrical container, and the urging means for urging the piston relative to the cylindrical container. With this arrangement, as the fluid is injected through the check valve, the piston is pushed in such a direction as to contract the urging means, whereby the fluid is stored in the fluid storing chamber defined by the cylindrical container and the piston. When the stop valve is opened, the fluid in the fluid storing chamber flows out therefrom via the regulating means, as the piston is moved backward due to a restoring force of the urging means.

Preferably, the contraction valve means is operative to flow the fluid out of the cuff through the pilot balloon.

In the above arrangement, since the connecting part is comprised of the pilot balloon, and the contraction valve means which is located at a specified position relative to the pilot balloon, the operator can check the inflated state of the cuff of the medical implement by touching. Further, this arrangement enables the operator to promptly draw the air out of the cuff through the contraction valve means.

Another aspect of the present invention provides a bronchus closing implement, as defined in claim 5.

In the bronchus closing implement equipped with the inflation assisting implement of the medical implement with cuff, the fluid injected through the check valve is temporarily stored in the cuff inflating means in a state that the cuff provided at the patient-side end of the catheter main body is located at the predetermined closing site in the patient's bronchus. When the stop valve is opened, the fluid stored in the cuff inflating means is injected into the cuff through the regulating means, the ramifying part, the ramifying-side side hole, the second lumen, and the cuff-side side hole, thereby inflating the cuff. Since the flow rate of the fluid is adjusted to an appropriate level by the regulating means, the healthcare worker can inflate the cuff with a simplified operation, thereby preventing excessive inflation of the cuff.

Furthermore, the first lumen extending through the catheter main body has the open end at the patient-side after the bronchus is closed. Thereby, the operator is allowed to suck the air remaining in the closed lung, and the body fluid adhering to a region near the lung, as well as supplying oxygen through the suction port provided at the operator-side of the catheter main body.

Moreover, since the ramifying part includes the pilot balloon, and the contraction valve means which is located at a specified position relative to the pilot balloon, the healthcare worker can check the inflated state of the cuff by touching, and promptly draw the air out of the cuff by manipulating the contraction valve means.

Preferably, the cuff inflating means of the bronchus closing implement includes a retractable container, and a cover member which protects the retractable container and regulates a chargeable amount of the liquid in the retractable container.

In the above arrangement, since the cuff inflating means is comprised of the retractable container, the fluid injected through the check valve is temporarily stored in the retractable container. When the stop valve is opened, the fluid flows out of the retractable container via the regulating means due to a contraction force of the retractable container. Further, since the cuff inflating means has the cover member, the retractable container is protected from external influence. Also, since the flow rate of the fluid to be charged into the retractable container is controlled, excessive injection of the fluid into the cuff is prevented.

The cuff inflating means may include a cylindrical container, a piston which is slidably movable in the cylindrical container in such a manner that an expandable and contractible fluid storing chamber is defined in the cylindrical container, and urging means which urges the piston in such a direction as to reduce the volume of the fluid storing chamber.

In the above arrangement, the cuff inflating means is comprised of the cylindrical container, the piston which is slidably movable in the cylindrical container, and the urging means which urges the piston and the gasket relative to the cylindrical container. With this arrangement, as the fluid is injected into the cylindrical container through the check valve, the piston is moved in such a direction as to contract the urging means, with the result that the fluid is stored in the fluid storing chamber defined by the cylindrical container and the piston. When the stop valve is opened, the fluid in the fluid storing chamber flows out therefrom via the regulating means, as the piston is moved backward due to a restoring force of the urging means.

### BRIEF DESCRIPTION OF THE DRAWINGS

FIG. 1 is an exploded perspective view showing an inflation assisting implement used in a medical implement with cuff (balloon) according to a first embodiment of the present invention.
FIG. 2A is an exploded perspective view of a check valve unit shown in FIG. 1.
FIG. 2B is a cross-sectional view showing an assembled state of the check valve unit shown in FIG. 1
FIG. 2C is a cross-sectional view showing a state of use of the assembled check valve unit shown in FIG. 1.
FIG. 3A is a cross-sectional view showing a state before use of the assembled inflation assisting implement shown in FIG. 1.
FIG. 3B is a cross-sectional view showing a state of use of the assembled inflation assisting implement shown in FIG. 1.
FIG. 4 is a partially cutaway perspective view of an endotracheal tube used in cooperation with the inventive inflation assisting implement.
FIG. 5 is an illustration showing how the endotracheal tube is applied to a patient.
FIG. 6 is a partially cutaway cross-sectional view of an inflation assisting implement according to a second embodiment of the present invention.
FIG. 7 is a cross-sectional view showing an assembled state of an inflation assisting implement according to a third embodiment of the present invention.
FIG. 8 is a partially cutaway external view of a bronchus closing implement according to an embodiment of the present invention.
FIG. 9 is a partially cross-sectional view showing an assembled state of the bronchus closing implement shown in FIG. 8.
FIG. 10 is an illustration showing how the bronchus closing implement shown in FIG. 8 is applied to a patient.

### BEST MODE FOR CARRYING OUT THE INVENTION

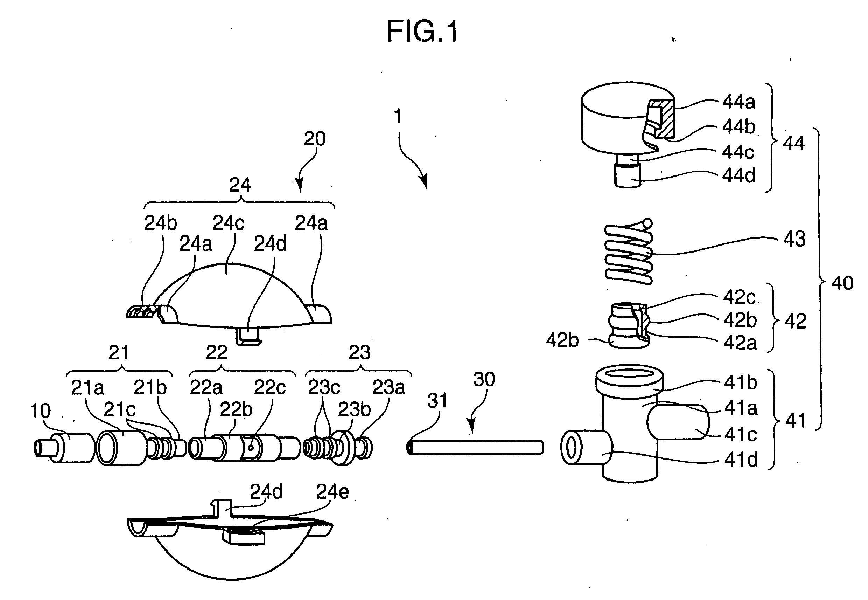

FIG. 1 is an exploded perspective view of an inflation assisting implement used in a medical implement with cuff (balloon) (hereinafter, called as "inflation assisting implement") according to a first embodiment of the present invention. As shown in the drawings, the inflation assisting implement 1 includes a check valve unit 10 connectable with an injection tube or the like, cuff inflating means 20 to be connected with the check valve unit 10, regulating means 30 to be connected with the cuff inflating means 20 for regulating outflow of a gas or a liquid from the cuff inflating means 20, and a stop valve unit 40 to be connected with the regulating means 30 for allowing outflow of the gas or the liquid from the regulating means 30.

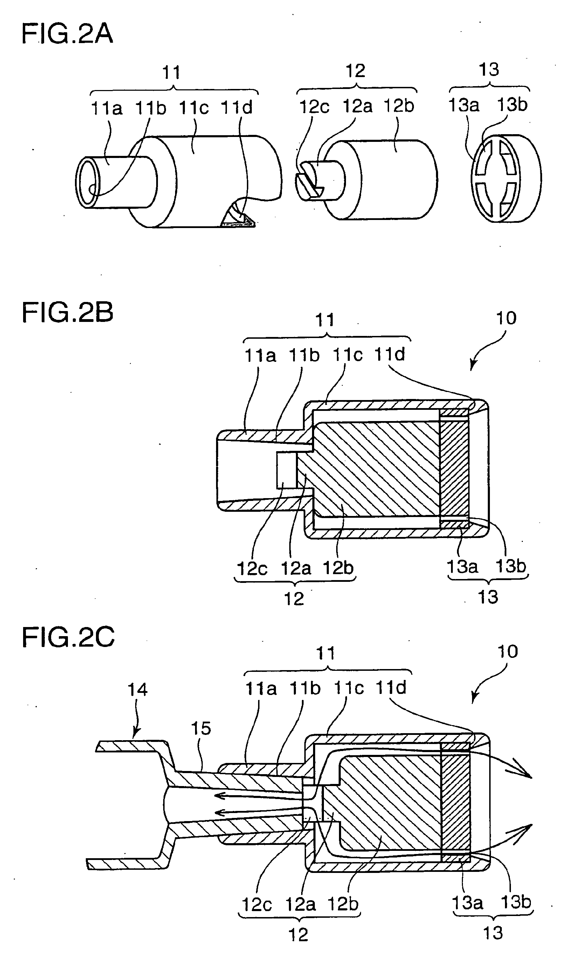

FIGS 2A to 2C are illustrations showing the check valve unit 10 according to the first embodiment of the present invention. Specifically, FIG. 2A is an exploded perspective view of the check valve unit 10, FIG. 2B is a cross-sectional view showing an assembled state of the check valve unit 10, and FIG. 2C is a cross-sectional view showing a state of use of the assembled check valve unit 10. As shown in FIG. 2A, the check valve unit 10 has a check valve main body 11, a valve 12, and a retainer 13.

The check valve main body 11 is made of a material such as a synthetic resin, a metal, or glass, and includes a hollow small-diametrical cylinder part 11a, a female taper part 11b constituting the inner wall of the small-diametrical cylinder part 11 a, and a hollow large-diametrical cylinder part 11c continuing from the small-diametrical cylinder part 11a. An engaging projection 11d is formed at an inner surface of the large-diametrical cylinder part 11c for engaging with the retainer 13, which will be described later.

The valve 12 is made of a synthetic resin having elasticity, or a like material, and includes a solid small-diametrical cylinder part 12a, a solid large-diametrical cylinder part 12b concentrically formed with the small-diametrical cylinder part 12a, and a cutaway part 12c formed in a diametrical direction of the small-diametrical cylinder part 12a at one end thereof.

The retainer 13 is made of a material such as a synthetic resin, a metal, or glass, and includes a disc part 13a, and plural channels 13b formed in an axial direction of the disc part 13a substantially over the length thereof.

The check valve unit 10 having the above construction is assembled, as shown in FIG. 2B, by inserting the valve 12 and the retainer 13 in the check valve main body 11, and engaging the bottom surface of the retainer 13 with the engaging projection 11d of the check valve main body 11. Thereby, the bottom surface of the large-diametrical cylinder part 12b of the valve 12 is supported on the upper surface of the retainer 13, and the upper surface of the large-diametrical cylinder part 12b is supported on the top surface of the large-diametrical cylinder part 11 c of the check valve main body 11. The entire length of the large-diametrical cylinder part 12b in the assembled state is set slightly longer than the distance from the upper surface of the retainer 13 to the top surface of the large-diametrical cylinder part 11c. With this arrangement, the space defined by the top surface of the large-diametrical cylinder part 11c and the upper surface of the large-diametrical cylinder part 12b is securely brought to an airtight state. Further, in this state, the small-diametrical cylinder part 12a of the valve 12 is housed in the female taper part 11b of the check valve main body 11.

The check valve unit 10 in the assembled state is brought to a released state by inserting a male taper part 15 of an injection tube 14 into the female taper part 11b of the check valve main body 11, as shown in FIG. 2C. Specifically, as the male taper part 15 is received into the female taper part 11b, a lead end of the male taper part 15 pushes the top surface of the small-diametrical cylinder part 12a of the valve 12, thereby elastically contracting the valve 12. As a result, the airtight state of the space defined by the top surface of the large-diametrical cylinder part 11c and the upper surface of the large-diametrical cylinder part 12b is released, thereby allowing a fluid to flow from the injection tube 14 in the directions shown by the rightward arrows in FIG. 2C via the valve 12 and the channels 13b of the retainer 13. Further, as the airtight state is released, the check valve unit 10 is restored to the state as shown in FIG. 2B due to an elastic restoring force of the valve 12 in response to detachment of the injection tube 14, whereby the check valve unit 10 is brought to a closed state.

The cuff inflating means 20 includes, as shown in FIG. 1, a valve nipple 21, a retractable container 22, a nipple 23, and a cover member 24.

The valve nipple 21 is made of a material such as a synthetic resin, a metal, or glass, and includes a cylindrical housing part 21a, a cylindrical receiving part 21b continuing from the housing part 21 a, and an engaging rib21 c formed around the outer surface of the receiving part 21b. The check valve unit 10 is air-tightly fixed to the valve nipple 21 by inserting the check valve unit 10 in the housing part 21 a and connecting the parts by an adhesive.

The retractable container 22 is made of a synthetic resin having retractility, or the like, and includes a tubular container main body 22a, an inflation part 22b, and a through-hole 22c for communicating the interior and the exterior of the container main body 22a The container main body 22a has an inner diameter substantially equal to the outer diameter of the receiving part 21b of the valve nipple 21. The through-hole 22c is formed substantially near the middle in the axial direction of the container main body 22a. The opposite ends of the inflation part 22b are fixed to the container main body 22a by an adhesive in a state that the inflation part 22b covers the through-hole 22c near the middle in the axial direction of the container main body 22a.

The nipple 23 is made of a material such as a synthetic resin, a metal, or glass, and includes a cylindrical nipple main body 23a, a receiving part 23b, and an engaging rib 23c formed around the outer surface of the receiving part 23b. The regulating means 30 and the nipple 23 are air-tightly fixed to each other by inserting the regulating means 30 into the nipple main body 23a and connecting the parts by an adhesive.

The cover member 24 is made of a material such as a synthetic resin, a metal, or glass, and is constructed by assembling a pair of hemi-parts of a globular body. Each hemi-part of the cover member 24 includes a pair of opposing hemi-cylindrical fixing parts 24a, an engaging groove 24b formed in each of the fixing parts 24a, a hemi-spherical container-like protecting part 24c, an engaging pawl 24d to be engaged with an engaging hole 24e of the counterpart hemi-part of the cover member 24, and the engaging hole 24e. Each of the engaging grooves 24b of the fixing parts 24a has such dimensions as to engage the corresponding engaging rib 21c of the valve nipple 21 and the engaging rib 23c of the nipple 23.

The cuff inflating means 20 having the above construction is assembled by: inserting the receiving part 21b of the valve nipple 21, and the receiving part 23b of the nipple 23 through the opposite ends of the container main body 22a of the retractable container 22, respectively in a state that the check valve unit 10 is air-tightly and adhesively fixed to the valve nipple 21; housing the receiving parts 21b and 23b in the cover member 24 by closing the hemi-parts of the cover member 24 to each other, and fixing the hemi-parts to each other by engagement of the engaging pawls 24d in the counterpart engaging holes 24e of the cover member 24. In this state, the engaging rib 21c of the valve nipple 21 and the engaging rib 23c of the nipple 23 are fitted in the corresponding engaging grooves 24b of the cover member 24 with the opposite ends of the container main body 22a of the retractable container 22 being interposed therebetween. In this way, the valve nipple 21 and the nipple 23 are axially fixed to the cover member 24, while keeping the airtight state of the retractable container 22.

The regulating means 30 is made of a material such as a synthetic resin, a metal, or glass, and is formed with pores 31. The pores 31 are formed to regulate the flow rate of a fluid such as a gas or a liquid by exerting a flow resistance to the fluid when the gas or the liquid charged in the retractable container 22 is passed through the regulating means 30.

The regulating means 30 may be a known flow rate regulator as disclosed in, for example, Japanese Unexamined Patent Publication No. 2000-14777. With such an arrangement, the flow rate of the gas or the liquid charged in the retractable container 22 can be selectively adjusted to an appropriate level.

The stop valve unit 40 includes a stop valve main body 41, a valve plug 42, a compression spring 43, and a pusher 44.

The stop valve main body 41 is made of a synthetic resin, a metal, or glass, and includes a cylindrical housing part 41a with one end thereof closed, an engaging flange 41b formed around the outer surface of the open end of the housing part 41 a, a connecting part 41 c extending radially outwardly from a side surface of the housing part 41 a, and a cylindrical connecting tube 41 d extending radially outwardly from the side surface of the housing part 41a in a direction opposite to the extending direction of the connecting part 41c. The connecting part 41c has a size substantially equal to the size of the male taper part 15 of the injection tube 14. Further, the connecting tube 41d has an inner diameter slightly larger than the outer dimension of the controlling means 30. The controlling means 30 is air-tightly fixed to the stop valve main body 41 by inserting the controlling means 30 in the connecting tube 41d and connecting the parts by an adhesion.

The valve plug 42 is made of a synthetic resin having elasticity, or a like material, and includes a cylindrical valve plug main body 42a with one end thereof closed, two seal parts 42b each formed over the outer surface of the valve plug main body 42a, and an engaging projection 42c having an inner diameter slightly smaller than the inner diameter of the valve plug main body 42a. The seal part 42b has a size slightly larger than the inner diameter of the housing part 41a of the stop valve main body 41. With this arrangement, when the valve plug 42 is received in the housing part 41a, the entire outer circumference of each of the seal parts 42b is fitted on the inner surface of the housing part 41a, thereby securing the airtight state of the stop valve unit 40.

The pusher 44 is made of a material such as a synthetic resin, a metal, or glass, and includes a cylindrical pusher main body 44a with one end thereof closed, an engaging flange 44b which is formed at an open end of the pusher 44 and has an inner diameter slightly smaller than the size of the pusher main body 44a, a solid cylindrical stem 44c concentrically formed with the pusher main body 44a, and an engaging projection 44d extending from the stem 44c at the opposite end to the pusher main body 44a.

The stop valve unit 40 having the above construction is assembled by inserting the valve plug 42 in the stop valve main body 41 with the compression spring 43 being interposed therebetween in a state that the stem 44c of the pusher 44 is received in the valve plug 42 and the controlling means 30 is connected with the stop valve main body 41. Thereby, the valve plug 42 is kept from being slipped off from the pusher 44 and is fixed to the pusher 44 by engagement of the engaging projection 42c with the engaging projection 44d of the pusher 44. The compression spring 43 has an urging force to retract the top surface of the stop valve main body 41 (the side of the engaging flange 41b) and the bottom surface of the pusher main body 44a of the pusher 44 away from each other. However, the pusher 44 and the stop valve main body 41 are fixed to predetermined respective positions within an allowable stroke by engagement of the engaging flange 44b of the pusher 44 and the engaging flange 41b of the stop valve main body 41.

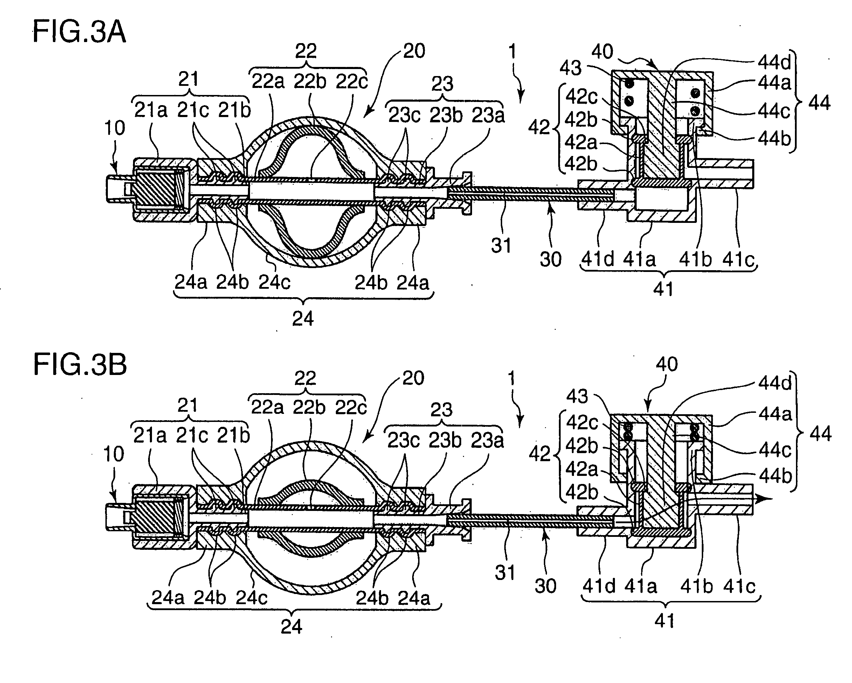

FIGS. 3A and 3B are cross-sectional views showing an assembled state of the inflation assisting implement 1. FIG. 3A shows a state before use, and FIG. 3B shows a state in use. In the following, an exemplified use of the inflation assisting implement is described as a first embodiment of the present invention.

FIG. 3A shows a state that a fluid such as a gas or a liquid of a predetermined volume is charged in the inflation assisting implement through the check valve unit 10 by an injection tube or the like. The gas or the liquid charged by the injection tube or the like is passed through the valve nipple 21, and reaches the stop valve main body 41 through the nipple 23 and the regulating means 30. Since the inner surface of the stop valve main body 41 and the seal parts 42b of the valve plug 42 are fixed to each other in the airtight state, the flow is substantially blocked thereat. As a result, the gas or the liquid is forced to flow into the inflation part 22b through the through-hole 22c of the retractable container 22, and is stored in the inflation part 22b.

The inner volume of the cover member 24 is defined in such a manner that an allowable storage of the gas or the liquid in the inflation part 22b is restricted by the protecting part 24c.

FIG. 3B shows a state that the pusher 44 is pushed downward from the state shown in FIG. 3A. When the inflation assisting implement 1 is brought to the state as shown in FIG. 3B, the connecting part 41 d and the connecting part 41 c of the stop valve main body 41 are located between the two seal parts 42b of the valve plug 42. Thereby, the gas or the liquid passed through the controlling portion flows in the direction as shown by the arrow in FIG. 3B through the connecting tube 41d, around the outer periphery of the valve plug main body 42a, and the connecting part 41 c.

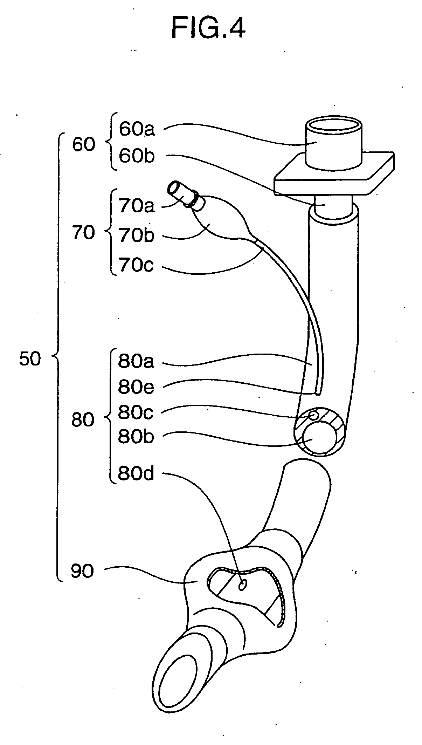

Next, a manner as to how the inflation assisting implement 1 as the first embodiment is used in cooperation with a medical implement with cuff (balloon) is described referring to FIG. 4 showing an endotracheal tube.

FIG. 4 is a partially cutaway perspective view of a generally available endotracheal tube. The endotrachael tube 50 includes a connector 60 connectable with a respirator, an injection portion 70 for inflating a cuff, a tubular portion 80 to be inserted in the trachea of a patient, and the cuff 90 for blocking the trachea of the patient.

The connector 60 has a cylindrical shape in its entirety, and includes a connecting part 60a to be connected with a respirator, and a connecting tube 60b capable of securing airtightness by being tightly fitted in a main body 80a of the tubular portion 80, which will be described later.

The injection portion 70 includes a check valve 70a having a substantially similar arrangement as the check valve unit shown in FIGS. 2A through 2C, a container-like pilot balloon 70b having openings at opposite ends thereof for allowing an operator to check an inflated state of the cuff by touching, and a connecting tube 70c to be connected with the tubular portion 80. The pilot balloon 70b has the one end thereof fixed to the check valve 70a by an adhesion, and the other end thereof being fixed to the connecting tube 70c by an adhesion, thereby securing an airtight state of the pilot balloon 70b.

The tubular portion 80 has the tubular main body 80a, a first hole 80b formed in the tubular main body 80a over the entire length of the tubular portion 80, a second hole 80c partially formed in the tubular main body 80a, a first side hole 80d formed in the tubular main body 80a near the patient-side for communication with the second hole 80c, and a second side hole 80e formed in the tubular main body 80a near the other end of the tubular main body 80a for communication with the second hole 80c. The connecting tube 70c and the tubular portion 80 are air-tightly connected with each other by an adhesive at a location corresponding to the second side hole 80e.

The cuff 90 is air-tightly fixed to the tubular portion 80 by an adhesive in such a manner that the opposite ends of the cuff 90 cover the first side hole 80d.

In the endotracheal tube 50 having the above construction, the gas injected through the check valve 70a is drawn into the cuff 90 through the pilot balloon 70b, the connecting tube 70c, the second side hole 80e, the second hole 80c, and the first side hole 80d. The gas is drawn in and out of the patient-side end of the tubular main body 80a through the connecting tube 60b and the first hole 80b by connecting the connecting part 60a with the respirator.

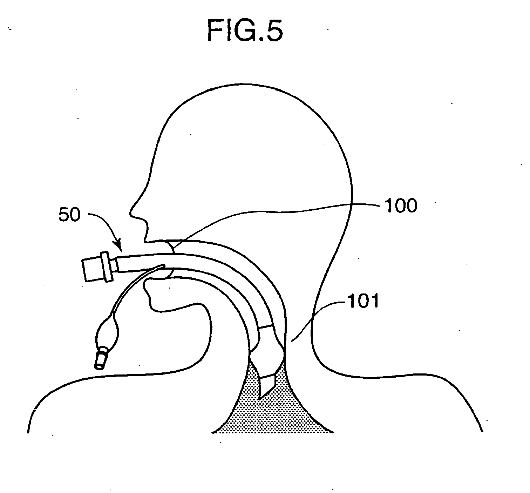

FIG. 5 is an illustration showing a state as to how the endotracheal tube 50 is applied to a patient. When the endotracheal tube 50 is inserted in the patient's body through a mouth 100, and the cuff is inflated in a trachea 101, forced respiration is initiated by the respirator at a site (the hatched region in FIG. 5) below the trachea 101.

In using the inflation assisting implement 1 in cooperation with the endotracheal tube 50, a healthcare worker connects the inflation assisting tube 1 with the check valve 70a in a state that a gas has been injected in the inflation assisting tube 1, in place of an injection tube. When the healthcare worker pushes the pusher 44 downward, the gas flows into the cuff 90 at a flow rate controlled by the regulating means 30 to inflate the cuff, whereby forced respiration is started.

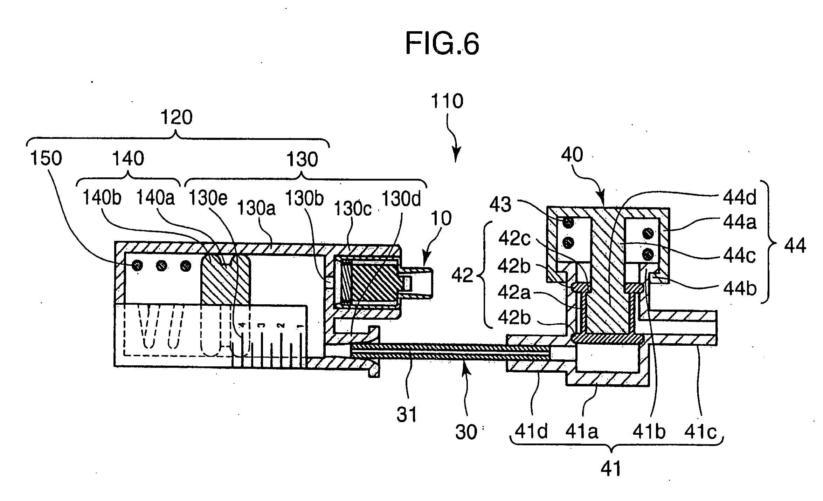

FIG. 6 is a partially cutaway cross-sectional view showing an assembled state of an inflation assisting implement as a second embodiment of the present invention. As shown in FIG. 6, the inflation assisting implement 110 includes a check valve unit 10 connectable with an injection tube or the like, cuff inflating means 120 to be connected with the check valve unit 10, regulating means 30 to be connected with the cuff inflating means 120 for regulating the outflow of a gas or a liquid from the cuff inflating means 120, and a stop valve unit 40 to be connected with the regulating means 30 for allowing outflow of the gas or the liquid from the regulating means 30. Since the arrangements of the check valve unit 10, the regulating means 30, and the stop valve unit 40 in the second embodiment are identical to those in the first embodiment, description thereof will be omitted herein.

The cuff inflating means 120 includes a cylindrical container 130 for storing the injected gas or liquid therein, a piston 140 for storing the gas or the liquid for outflow of the gas or the liquid, and a compression spring 150 which is urged in such a direction as to drive a gasket 140 for outflow of the gas or the liquid.

The cylindrical container 130 is made of a material having transparency, such as a synthetic resin or glass, and includes a cylindrical container main body 130a with the opposite ends thereof closed, a communication hole 130b extending through a wall portion corresponding to one of the closed ends of the container main body 130a, a cylindrical housing part 130c extending from the container main body 130a through the communication hole 130b, a cylindrical connecting part 130d communicable with the container main body 130a, and a calibration part 130e formed on the outer surface of the container main body 130a for allowing an operator to presumably calculate the stored amount of the gas or the liquid based on a relative position of the gas or the liquid to the gasket. The check valve unit 10 is air-tightly fixed to the container 130 by inserting the check valve unit 10 in the housing part 130c and connecting the parts with an adhesive. The container 130 is air-tightly fixed to the regulating means 30 by inserting the regulating means 30 in the connecting part 130d and connecting the parts with an adhesive.

The piston 140 is made of a synthetic resin having elasticity, or the like, and includes a solid cylindrical main body 140a, and a seal part 140b having an outer dimension larger than the dimension of the main body 140a. The piston 140 is received in the cylindrical container 130, and has such a size that the outer surface of the seal part 14b is slidably movable to the inner surface of the cylindrical container 130 while keeping the air-tight state of the container 130.

The compression spring 150 is made of a metal or the like, with an end thereof supported on the closed wall corresponding to a lower end (left side in FIG. 6) of the cylindrical container 130, and the other end thereof supported on a lower end (left side in FIG. 6) of the piston 140. Further, the compression spring 150 is so configured that a compression force is exerted to move an upper end (right side in FIG. 6) of the piston 140 toward the closed wall corresponding to an upper end (right side in FIG. 6) of the cylindrical container 130 in an initial state that the compression spring 150 is mounted in the cylindrical container 130.

In the thus constructed inflation assisting implement 110, when a gas or a liquid is injected in the inflation assisting implement 110 through the check valve unit 10, and is drawn into the cylindrical container 130 through the communication hole 130b, the piston 140 is retracted. As a result, the gas or the liquid is stored in the cylindrical container 130. At this time, since the operator can view the behavior of the piston 140 through the transparent cylindrical container 130, the operator can confirm the stored amount of the gas or the liquid based on the position of the gas or the liquid relative to the gasket on the calibration part 130e. The compression spring 150 is contracted, as the piston 140 is retracted.

In the above state, when the healthcare worker pushes the pusher 44 downward, the stop valve unit 40 is released, whereby the gas or the liquid in the cylindrical container 130 flows out therefrom at a predetermined fluid rate controlled by the regulating means 30.

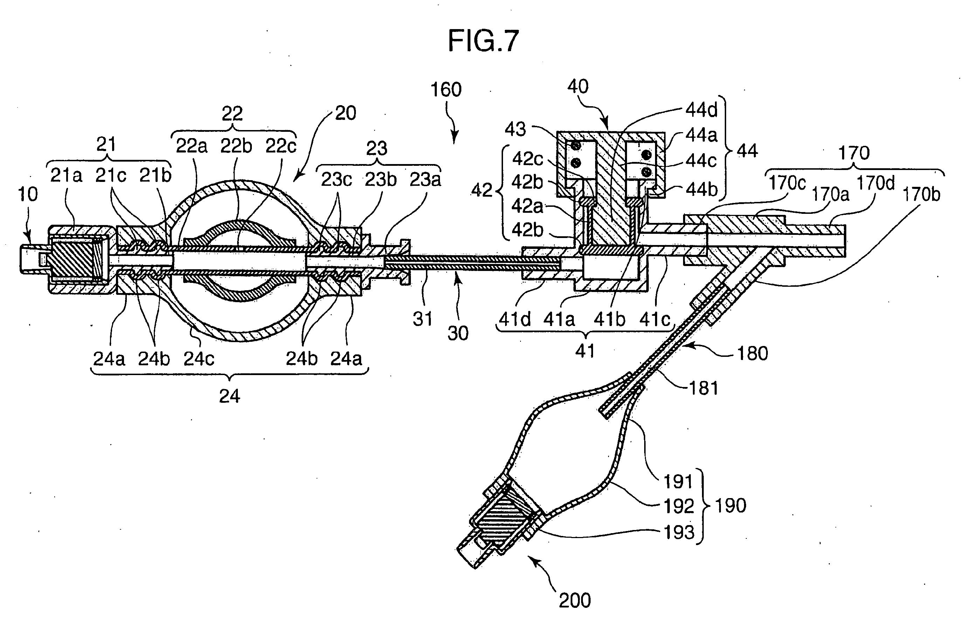

FIG. 7 is a cross-sectional view showing an assembled state of an inflation assisting implement as a third embodiment of the present invention. The inflation assisting implement 160 comprises elements corresponding to the elements in the first embodiment, namely, a check valve unit 10 connectable with an injection tube or the like, cuff inflating means 20 to be connected with the check valve unit 10, regulating means 30 to be connected with the cuff inflating means 20 for regulating outflow of the gas or the liquid from the cuff inflating means 20, and a stop valve unit 40 to be connected with the regulating means 30 for allowing outflow of the gas or the liquid from the regulating means 30. The inflation assisting implement 160 further includes a ramifying part 170 to be connected with the stop valve unit 40 for distributing the gas or the liquid in two directions, a connecting tube 180 to be connected with an end of the ramifying part 170, a pilot balloon 190 to be connected with the connecting tube 180 for allowing an operator to check an inflated state of the cuff by touching, and a contraction check valve unit 200 to be connected with the pilot balloon 190.

The ramifying part 170 is made of a material such as a synthetic resin, a metal, or glass, and includes a cylindrical primary tube 170a, a cylindrical secondary tube 170b diverging from the primary tube 170a at a predetermined angle, a female taper part 170c to be connected with the stop valve unit 40 in the primary tube 170a, and a male taper part 170d to be connected with the check valve 70a of the endotracheal tube 50, and the like.

The connecting tube 180 is made of a material such as a synthetic resin or glass, and is formed with pores 181 having a size slightly larger than the size of the pores 31 of the regulating means 30. The connecting tube 180 is air-tightly fixed to the ramifying part 170 by inserting an end of the connecting tube 180 in the secondary tube 170b of the ramifying part 170 and connecting the parts by an adhesive.

The pilot balloon 190 is made of a synthetic resin having elasticity, or the like, and includes a small-diametrical end 191 to be connected with the other end of the connecting tube 180, a detecting part 192 for allowing an operator to check an inflated state of the cuff by touching, and a large-diametrical end 193 to be connected with the contraction check valve unit 200.

Generally, the contraction check valve unit 200 is the one as shown in FIGS. 2A through 2C. The contraction check valve unit 200 is air-tightly fixed to the pilot balloon 190 by inserting the contraction check valve unit 200 in the large-diametrical part 193 of the pilot balloon 190 and connecting the parts by an adhesive.

The inflation assisting implement 160 having the above construction is used by connecting the inflation assisting implement 160 with the check valve 70a of the endotracheal tube 50 in a state that a gas has been injected in the implement 160. When a healthcare worker pushes the pusher 44 downward, the gas flows into the cuff 90 at a predetermined flow rate controlled by the regulating means 30 to inflate the cuff 90, whereby forced respiration is initiated.

Upon confirming that the forced respiration is no longer necessary, the healthcare worker connects an injection tube or the like with the contraction check valve unit 200 for sucking the air. Specifically, since the stop valve unit 40 is normally closed, the air in the cuff can be promptly drawn out by connecting the injection tube with the contraction check valve unit 200.

When the cuff 90 is inflated, the operator can check the inflated state of the cuff 90 by touching the detecting part 192 of the pilot balloon 190. This arrangement is advantageous in the case where an element corresponding to the pilot balloon 70b is not provided in the endotracheal tube 50.

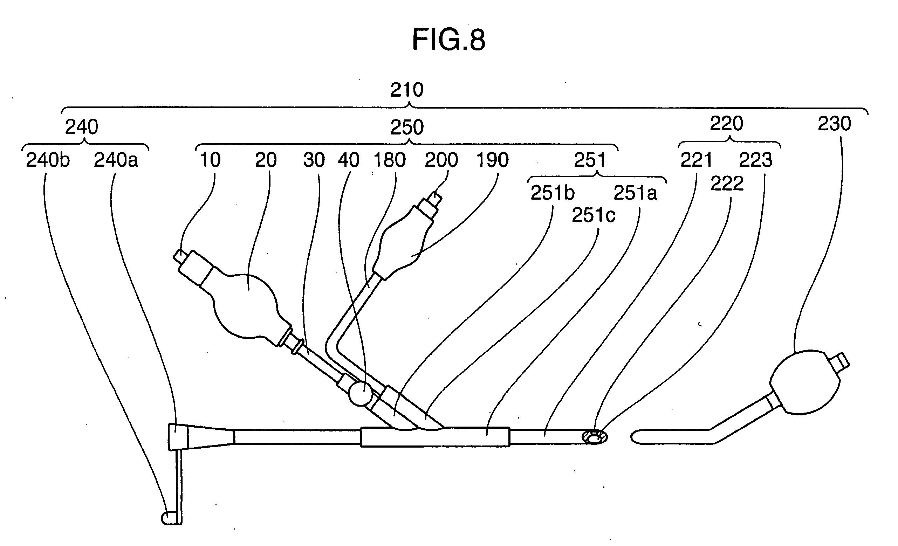

FIG. 8 is a partially cutaway external view of a bronchus closing implement 210 provided with an inflation assisting implement. The bronchus closing implement 210 includes a catheter main body 220 to be inserted in the bronchus of a patient, a bronchus cuff 230 which is fixed to an end of the catheter main body 220 corresponding to the patient side for blocking the bronchus, a suction unit 240 which is fixed to the other end of the catheter main body 220 corresponding to the operator side for sucking the body fluid in the bronchus, and an inflation assisting unit 250 for assisting inflation of the bronchus cuff 230.

The catheter main body 220 is made of a synthetic resin having elasticity, or the like, and includes a tubular catheter 221, a small-diametrical second lumen 222 partially extending through the entire length of the catheter 221, and a large-diametrical first lumen 223 substantially extending through the entire length of the catheter 221.

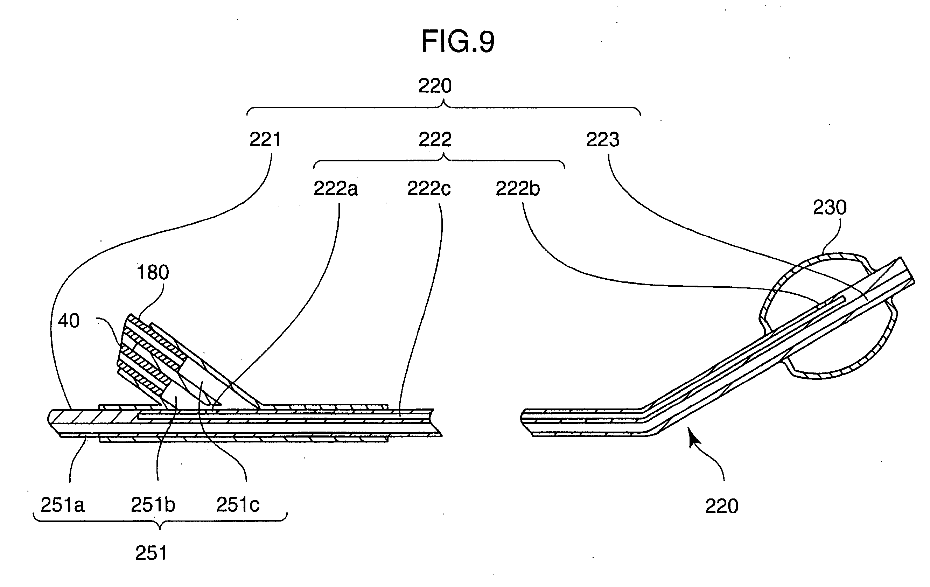

FIG. 9 is a partially cross-sectional view showing an assembled state of the bronchus closing implement 210. The second lumen 222 includes a ramifying-side side hole 222a for communicating with the exterior of the catheter 221 at the operator-side, a cuff-side side hole 222b for communicating with the exterior of the catheter 221 at the patient-side, and a communication channel 222c for communicating with the ramifying-side side hole 222a and the cuff-side side hole 222b. The ramifying-side side hole 222a is configured such that a first tubular part 251b and a second tubular part 251b are communicable with each other inside a ramifying tube 251, which will be described later. The cuff-side side hole 222b is so configured as to be communicable with the interior of the bronchus cuff 230.

The bronchus cuff 230 is made of a synthetic resin having elasticity, or the like, and is securely connected with the outer surface of the catheter main body 220 by an adhesive at longitudinal opposite ends of the bronchus cuff 230.

The suction unit 240 is made of a synthetic resin having elasticity, or the like, and includes a conical trapezoidal suction port 240a connectable with an injection tube or the like, and a plug member 240b adapted for closing the suction port 240a. The suction unit 240 is air-tightly and securely connected with the catheter main body 220 by connecting an end of the suction unit 240 with the catheter main body 220 by an adhesive.

The inflation assisting unit 250 includes a check valve unit 10 connectable with an injection tube or the like, cuffinflating means 20, regulating means 30 to be connected with the cuff inflating means 20 for regulating the outflow of a gas or a liquid from the cuff inflating means 20, a stop valve unit 40 to be connected with the regulating means 30 for allowing outflow of the gas or the liquid from the regulating means 30, the ramifying tube 251 to be connected with the stop valve unit 40 for distributing the gas or the liquid, a connecting tube 180 to be connected with an end of the ramifying tube 251, a pilot balloon 190 to be connected with the connecting tube 180 for allowing an operator to check an inflated state of the cuff 230 by touching, and a contraction check valve unit 200 to be connected with the pilot balloon 190. Description on the elements which are identical to those in the third embodiment will be omitted herein.

The ramifying tube 251 is made of a material such as a synthetic resin, a metal, or glass, and includes a cylindrical primary tube 251a capable of fittingly receiving the catheter main body 220, the first tubular part 251b to be fixed to the stop valve unit 40 by an adhesive, and the second tubular part 251c to be fixed to the connecting tube 180 by an adhesive. The catheter main body 220 is fitted in the primary tube 251a and is fixed at the longitudinal opposite ends thereof by an adhesive. In this arrangement, as shown in FIG. 9, the stop valve unit 40 and the connecting tube 180 are communicable with each other through the communication channel 222c further to the cuff-side side hole 222b via the ramifying-side side hole 222a of the catheter main body 220.

The bronchus closing implement 210 having the above construction is applied to the patient in forced respiration, as shown in FIG. 5, for example, in a case where surgery of partially removing the left lung of the patient is required.

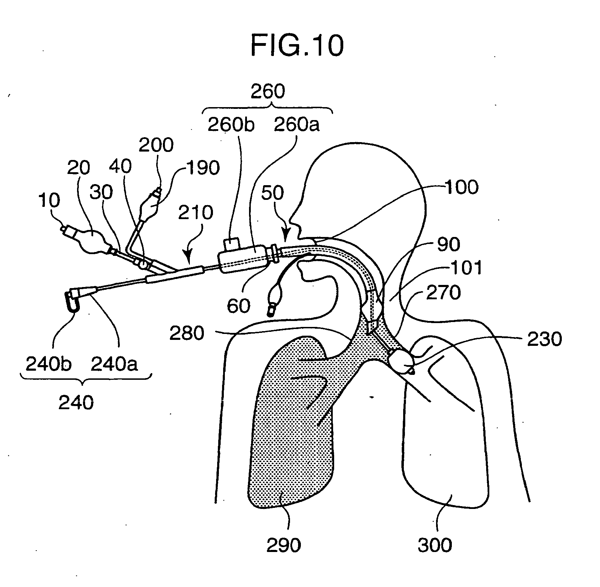

FIG. 10 is an illustration showing a state as to how the bronchus closing implement 210 is applied to a patient. Let it be assumed that the patient is in a condition that part of the left lung 300 is to be removed. Under such a condition, since forced respiration is required for the right lung 290 only, a healthcare worker temporarily disconnects the respirator from the connector 60 of the endotracheal tube 50 that has been mounted in the patient in forced respiration, and attaches an adaptor 260 to the connector 60.

The adaptor 260 includes an insertion tube 260a for guiding the bronchus closing implement 210 into the endotracheal tube while securing the airtight state of the bronchus closing implement 210, and a connecting part 260b to be connected with the respirator.

After being inserted into the adaptor 260, the bronchus closing implement 210 is guided into the patient's body along the first hole 80b (see FIG. 4) of the endotracheal tube 50. Generally, a healthcare worker inserts a bronchus endoscope (not shown) along with the bronchus closing implement 210, and inflates the bronchus cuff 230 by manipulating the stop valve unit 40 under direct viewable state of the region around a target left-side bronchus 270 through the bronchus endoscope in guiding the bronchus cuff 230 to the left-side bronchus 270 of the patient. Since the endotracheal region of the patient between the bronchus cuff 230 and the cuff 90 is blocked while the cuff 90 is inflated in the trachea 101, and the bronchus cuff 230 is inflated in the left-side bronchus 270, the gas from the respirator is drawn in and out of a right-side bronchus 280 and the right lung 290 through the endotracheal tube 50 (see the dotted region in FIG. 9).

The healthcare worker can check the inflated state of the bronchus cuff 230 by touching the pilot balloon 190. When the healthcare worker detaches the plug member 240b of the suction port 240a, he or she is allowed to suck the body fluid or the like in the bronchus therefrom by using an injection tube or the like, and also allowed to draw out the air remaining in the left lung 300. The air in the bronchus cuff 230 can be drawn out through the contraction check valve unit 200.

In the foregoing embodiment, a retractable container is used as the cuff inflating means 20 of the bronchus closing implement 210. Alternatively, the cuff inflating means 120 shown in FIG. 6 may be usable as the cuff inflating means of the bronchus closing implement.

### EXPLOITATION IN INDUSTRY

In the inventive inflation assisting implement used in a medical implement with cuff, the cuff is inflated by a simplified manipulation of the stop valve. This arrangement is advantageous in preventing excessive inflation of the cuff, and providing versatile use of the inflation assisting implement in cooperation with various medical implements with cuff.

Further, in the inventive bronchus closing implement, the cuff can be inflated with an easy operation of the stop valve by a healthcare worker. This arrangement is advantageous in preventing excessive inflation of the cuff in blocking the bronchus, even under a condition that the healthcare worker has to manipulate the bronchus endoscope or the like with his or her hand while manipulating the cuff, and accordingly, has to inflate the cuff with his or her other hand.

## Claims

1. An inflation assisting implement (160, 250) mountable on a medical implement with cuff (230) to inflate the cuff(230) by feeding a fluid to the cuff(230), the implement (160) comprising:
a check valve (10) connectable with injection means including an injection tube (14);
cuff inflating means (20, 120) which stores a fluid passing through the check valve (10) and is operative to flow out the fluid therefrom;
regulating means (30) which regulates the outflow of the fluid from the cuff inflating means (20, 120) to a predetermined flow rate;
a stop valve (40) which is normally closed and opened by a specified operation to flow out the fluid stored in the cuff inflating means (20,120); and
a connecting part (41c, 170, 251) which feeds the fluid out of the cuff inflating means (20, 120) to the medical implement with cuff(230), in response to manipulation of the stop valve (40);
**characterised in that** the connecting part (41 c, 170, 251) includes a ramifying part (170, 251) to be connected with the stop valve (40) for distributing the fluid in two directions, a pilot balloon (190) which allows an operator to check an inflated state of the cuff (230) by touching, and contraction valve means (200) which is operable to flow fluid out of the cuff (230) without passing through the regulating means (30).

2. The inflation assisting implement (160, 250) according to Claim 1, wherein the cuff inflating means (20) includes a retractable container (22), and a cover member (24) which protects the retractable container (22) and regulates a chargeable amount of the liquid in the retractable container (22).

3. The inflation assisting implement (160, 250) according to Claim 1, wherein the cuff inflating means (120) includes a cylindrical container (130), a piston (140) which is slidably movable in the cylindrical container (130) in such a manner that an expandable and contractible fluid storing chamber is defined in the cylindrical container (130), and urging means (150) which urges the piston (140) in such a direction as to reduce the volume of the fluid storing chamber.

4. The inflation assisting implement according to any one of claims 1 through 3, wherein the contraction valve (200) means is operative to flow the fluid out of the cuff(230) through the pilot balloon(190).

5. A bronchus closing implement (210) operative to openably close the bronchus of a patient, comprising:
a tubular catheter main body (220) having such a length as to reach a certain site of the patient's bronchus for blocking, the catheter main body (220) extending through a first lumen (223) over the entire length thereof, and extending through a second lumen (222) by a partial length thereof, the first lumen (223) having an open end on the side of the patient with respect to the catheter main body (220), the second lumen (222) having a cuff-side side hole (222b) in the vicinity of a patient-side end thereof, and a ramifying-side side hole (222a) in the vicinity of an operator-side end thereof;
a cuff (230) which is fixed to the catheter main body (220) in such a manner as to cover the cuff-side side hole (222b);
an inflation assisting implement (250) according to any one of claims 1 through 4, the inflation assisting implement (250) including the ramifying part (251) which is fixed to the catheter main body (220); and
a suction port (240a) provided at an operator-side of the catheter main body (220) and connectable with injection means including an injection tube (14);
wherein the ramifying part (251) extends by such a length as to cover the ramifying-side side hole (222a).

## Patentansprüche

1. Inflations-Unterstützungs-Mittel (160, 250) befestigbar in einer medizinischen Vorrichtung mit einer Manschette (230), um die Manschette (230) aufzublasen durch Einspeisen eines Fluids in die Manschette (230), wobei das Mittel (160) aufweist:
ein Verschlussventil (10), das mit einem Inflations-Mittel verbindbar ist, das eine Injektionsröhre (14) aufweist;
Manschetten-Inflations-Mittel (20, 120), das ein Fluid speichert, das durch das Verschlussventil (10) fließt, und das funktionsfähig ist, um das Fluid hieraus fließen zu lassen;
Regelmittel (30), das das Ausfließen des Fluids aus dem Manschetten-Inflations-Mittel (20, 120) in einer vorbestimmten Fliessrate reguliert;
ein Absperrventil (40), das normalerweise geschlossen ist und durch eine festgelegte Bedienung geöffnet wird, um das Fluid abfließen zu lassen, das in dem Manschetten-Inflations-Mittel (20, 120) gespeichert ist; und
einen verbindenden Teil (41c, 170, 251), der das Fluid aus dem Manschetten-Inflations-Mittel (20, 120) zu dem medizinischen Mittel mit der Manschette (230) einspeist, in Reaktion zu einer Bedienung des Absperrventils (40);
**dadurch gekennzeichnet, dass** der verbindende Teil (41c, 170, 251) einen verzweigenden Teil (170, 251) aufweist, der mit dem Absperrventil (40) zu verbinden ist, um das Fluid in zwei Richtungen zu verteilen, einen Pilot-Ballon (190) aufweist, der einem Bediener erlaubt, einen Inflations-Zustand der Manschette (230) durch Berühren zu prüfen, und ein Kontraktions-Ventil-Mittel (200) aufweist, das funktionsfähig ist, um Fluid aus der Manschette (230) ohne Passieren des Regelungsmittels (30) ausfließen zu lassen.

2. Inflations-Unterstützungs-Mittel (160, 250) nach Anspruch 1, wobei das Manschetten-Inflations-Mittel (20) einen einschiebbaren Behälter (22) aufweist, und ein Abdeckmittel (24) aufweist, das den einschiebbaren Behälter (22) schützt und das eine dosierbare Menge der Flüssigkeit in dem einschiebbaren Behälter (22) reguliert.

3. Inflations-Unterstützungs-Mittel (160, 250) nach Anspruch 1, wobei das Manschetten-Inflations-Mittel (120) einen zylindrischen Behälter (130), einen Kolben (140) und ein Drängmittel (150) aufweist, wobei der Kolben gleitend bewegbar in dem zylindrischen Behälter (130) ist, so dass eine expansionsfähige und kontraktionsfähige Fluidspeicherkammer in dem zylindrischen Behälter (130) definiert wird, wobei das Drängmittel (150) den Zylinder (140) in eine derartige Richtung drängt, um das Volumen der Fluidspeicherkammer zu reduzieren.

4. Inflations-Unterstützungs-Mittel nach einem der Ansprüche 1 bis 3, wobei das Kontraktions-Ventil-Mittel (200) funktionsfähig ist, um das Fluid aus der Manschette (230) durch den Pilot-Ballon (190) ausfließen zu lassen.

5. Bronchus-Verschluss-Vorrichtung (210), die funktionsfähig ist, um durch Öffnen den Bronchus eines Patienten zu schließen, aufweisend:
einen rohrförmigen Katheter-Hauptkörper (220), der eine derartige Länge aufweist, so dass ein bestimmter Ort des Bronchus eines Patienten zum Blockieren erreichbar ist, wobei sich der Katheter-Hauptkörper (220) durch ein erstes Lumen (223) über die gesamte Länge hiervon erstreckt, und sich durch ein zweites Lumen (222) über eine Teillänge hiervon erstreckt, wobei das erste Lumen (223) ein offenes Ende an der Seite des Patienten in Bezug auf den Katheter-Hauptkörper (220) hat, wobei das zweite Lumen (222) eine manschettenseitige Seitenöffnung (222b) in der Nähe des patientenseitigen Endes hiervon hat, und eine verzweigungsseitige Seitenöffnung (222a) in der Nähe eines bedienerseitigen Endes hiervon hat;
eine Manschette (230), die an dem Katheter-Hauptkörper (220) derart befestigt ist, um die manschettenseitige Seitenöffnung (222b) zu bedecken;
ein Inflations-Unterstützungs-Mittel (250) gemäß einem der Ansprüche 1 bis 4, wobei das Inflations-Unterstützungs-Mittel (250) den verzweigenden Teil (251) aufweist, der mit dem Katheter-Hauptkörper (220) verbunden ist; und
einen Sauganschluss (240a), die an einer Bedienerseite des Katheter-Hauptkörpers (220) bereitgestellt wird und mit einem Injektionsmittel, das eine Injektionsröhre (14) aufweist, verbindbar ist;
wobei der verzweigende Teil (251) sich derart in einer Länge erstreckt, um die verzweigungsseitige Seitenöffnung (222a) abzudecken.

## Revendications

1. Instrument d'aide au gonflage (160, 250) pouvant être monté sur un instrument médical doté d'un ballonnet (230), destiné à gonfler le ballonnet (230) en fournissant un fluide au ballonnet (230), l'instrument (160) comprenant :
un clapet anti retour (10) pouvant être connecté à des moyens d'injection qui comprennent un tube d'injection (14) ;
des moyens de gonflage de ballonnet (20, 120) qui stockent un fluide qui passe à travers le clapet anti-retour (10) et sont fonctionnels de façon à faire circuler le fluide à partir de là ;
des moyens de régulation (30) qui régulent la sortie du fluide en provenance des moyens de gonflage du ballonnet (20, 120) à un débit prédéterminé ;
une soupape d'arrêt (40) qui est normalement fermée et ouverte par une opération spécifiée de façon à faire circuler le fluide stocké dans les moyens de gonflage du ballonnet (20, 120) ; et
une partie connexion (41c, 170, 251) qui fournit le fluide en provenance des moyens de gonflage du ballonnet (20, 120) à l'instrument médical doté d'un ballonnet (230), en réponse à la manipulation de la soupape d'arrêt (40) ;
**caractérisé en ce que** la partie connexion (41c, 170, 251) comprend une partie ramification (170, 251) destinée à être connectée à la soupape d'arrêt (40) de façon à répartir le fluide dans deux directions, un ballon pilote (190) qui permet à un opérateur de vérifier par contact un état gonflé du ballonnet (230), et des moyens formant soupape de contraction (200) qui sont fonctionnels de façon à faire circuler le fluide hors du ballonnet (230) sans passer à travers les moyens de régulation (30).

2. Instrument d'aide au gonflage (160, 250) selon la revendication 1, dans lequel les moyens de gonflage du ballonnet (20) comprennent un contenant escamotable (22), et un élément formant couvercle (24) qui protège le contenant escamotable (22) et régule une quantité de liquide pouvant être chargée dans le contenant escamotable (22).

3. Instrument d'aide au gonflage (160, 250) selon revendication 1, dans lequel les moyens de gonflage du ballonnet (120) comprennent un contenant cylindrique (130), un piston (140) qui est mobile de façon coulissante dans le contenant cylindrique (130) de telle manière qu'une chambre de stockage de fluide pouvant s'expanser et se contracter soit définie dans le contenant cylindrique (130), et des moyens de poussée (150) qui poussent le piston (140) dans une direction telle que le volume de la chambre de stockage de fluide est réduit.

4. Instrument d'aide au gonflage selon l'une quelconque des revendications 1 à 3, dans lequel les moyens formant soupape de contraction (200) sont opérationnels de façon à faire s'écouler le fluide hors du ballonnet (230) à travers le ballon pilote (190).

5. Instrument de fermeture de bronche (210) opérationnel de façon à fermer de manière pouvant être ouverte une bronche d'un patient, comprenant :
un corps principal de cathéter tubulaire (220) qui présente une longueur qui permet d'atteindre un certain emplacement d'une bronche d'un patient de façon à la bloquer, le corps principal de cathéter (220) s'étendant à travers une première lumière (223) sur toute sa longueur, et s'étendant à travers une seconde lumière (222) sur une longueur partielle de celle-ci, la première lumière (223) présentant une extrémité ouverte du côté du patient par rapport au corps principal de cathéter (220), la seconde lumière (222) présentant un trou latéral du côté du ballonnet (222b) à proximité d'une extrémité du côté du patient, et un trou latéral du côté ramification (222a) à proximité d'une extrémité du côté d'un opérateur ;
un ballonnet (230) qui est fixé sur le corps principal de cathéter (220) de façon à couvrir le trou latéral du côté du ballonnet (222b) ;
un instrument d'aide au gonflage (250) selon l'une quelconque des revendications 1 à 4, l'instrument d'aide au gonflage (250) comprenant la partie ramification (251) qui est fixée sur le corps principal de cathéter (220) ; et
un orifice d'aspiration (240a) disposé au niveau du côté opérateur du corps principal de cathéter (220) et pouvant être connecté à des moyens d'injection qui comprennent un tube d'injection (14) ;
dans lequel la partie ramification (251) s'étend sur une longueur telle que le trou latéral du côté ramification (222a) est couvert.
